Europäisches Patentamt

European Patent Office

Office européen des brevets

(19)

(11) Numéro de publication : **0 438 329 A1**

# (12) DEMANDE DE BREVET EUROPEEN

(21) Numéro de dépôt : 91400030.2

(22) Date de dépôt : 09.01.91

(51) Int. Cl.[5] : **C07C 37/20, C07C 39/08**

(30) Priorité : 19.01.90 FR 9000814

(43) Date de publication de la demande :
24.07.91 Bulletin 91/30

(84) Etats contractants désignés :
AT BE CH DE DK ES FR GB GR IT LI LU NL SE

(71) Demandeur : RHONE-POULENC CHIMIE
25, quai Paul Doumer
F-92408 Courbevoie Cédex (FR)

(72) Inventeur : **Doussain, Claude**
**2, rue Louis Girardet**
**F-69190 Saint-Fons (FR)**
Inventeur : **Gubelmann, Michel**
**39, boulevard des Belges**
**F-69006 Lyon (FR)**
Inventeur : **Tirel, Philippe-Jean**
**40, boulevard Kennedy**
**F-69600 Oullins (FR)**

(74) Mandataire : **Dutruc-Rosset, Marie-Claude et**
**al**
**RHONE-POULENC CHIMIE Service Brevets**
**Chimie 25, Quai Paul Doumer**
**F-92408 Courbevoie Cédex (FR)**

(54) **Procédé de C-Alkylation de L'hydroquinone et de ses monoéthers.**

(57)     La présente invention concerne la C-alkylation de l'hydroquinone ou de ses monoéthers par réaction en phase vapeur avec un alcanal en présence d'un catalyseur solide.

Plus précisément, elle consiste en ce que l'on fait réagir en phase vapeur l'hydroquinone ou l'un de ses monoéthers d'alkyle de 1 à 4 atomes de carbone ou de cyclohexyle, avec un alcanal inférieur en présence d'au moins un oxyde métallique.

Parmi les alkylhydroquinones, un composé très intéressant est la méthylhydroquinone, servant à préparer le diacétate de méthylhydroquinone, composé utilisé comme monomère dans la synthèse de polymères thermotropes.

EP 0 438 329 A1

Jouve, 18, rue Saint-Denis, 75001 PARIS

# PROCEDE DE C-ALKYLATION DE L'HYDROQUINONE ET DE SES MONOETHERS

La présente invention concerne la C-alkylation de l'hydroquinone ou de ses monoéthers par réaction en phase vapeur avec un alcanal en présence d'un catalyseur solide.

Parmi les alkylhydroquinones, un composé très intéressant est la méthylhydroquinone, servant à préparer le diacétate de méthylhydroquinone, composé utilisé comme monomère dans la synthèse de polymères thermotropes.

De nombreuses voies d'accès à la méthylhydroquinone ont notamment été proposées.

Le brevet américain n° 2 041 593 décrit l'hydrolyse du chloro-4 méthyl-2 phénol en méthylhydroquinone, en milieu aqueux sodique.

Le brevet EP-A-0 041 441 décrit l'hydroxylation de l'orthocrésol dans le fluorure d'hydrogène à - 40°C, en présence de pentafluorure d'antimoine.

Le brevet américain n° 4 482 756 préconise l'oxydation de l'orthocrésol par l'oxygène, en présence de chlorure cuivrique dans l'acétonitrile.

La présente invention constitue en particulier une nouvelle voie de synthèse des alkylhydroquinones à partir de l'hydroquinone.

Plus précisément, elle consiste en ce que l'on fait réagir en phase vapeur l'hydroquinone, ou l'un de ses monoéthers d'alkyle de 1 à 4 atomes de carbone ou de cyclohexyle, avec un alcanal inférieur en présence d'au moins un oxyde métallique.

Lorsque l'on utilise un monoéther d'hydroquinone, il s'agit le plus souvent des monoéthers méthyliques, éthyliques, isopropyliques ou cyclohexyliques.

Dans le présent texte, par commodité le terme diphénol englobera l'hydroquinone et ses monoéthers définis précédemment.

Par alcanal inférieur on entend dans le présent texte les aldéhydes dérivant des alcanes ayant 1 à 4 atomes de carbone.

Comme tels alcanals, on peut citer le méthanal ou formaldéhyde, l'éthanal ou acétaldéhyde, le propanal, le butanal.

Parmi ces alcanals le méthanal est plus particulièrement utilisé.

Il peut être employé sous la forme de formaldéhyde gazeux, de solution aqueuse de formaldéhyde ou sous une forme solide polymérisée telle que le trioxanne-1,3,5.

Les oxydes métalliques servant de catalyseurs dans le procédé de l'invention peuvent être des oxydes acides, amphotères ou basiques, simples ou mixtes ou des mélanges de ces différents oxydes.

Ainsi parmi les oxydes généralement considérés comme acides ou amphotères, on peut citer comme oxydes simples l'oxyde de silicium (silice), l'oxyde d'aluminium (alumine), le dioxyde de titane, le dioxyde de zirconium, l'oxyde stannique, l'oxyde arsénieux, l'oxyde de plomb II, l'oxyde de bismuth III, l'oxyde de vanadium V, l'oxyde d'antimoine V, le dioxyde de thorium, l'oxyde de chrome VI, l'oxyde de molybdène VI.

Parmi les oxydes généralement considérés comme basiques, on peut citer plus particulièrement l'oxyde de magnésium, l'oxyde de lanthane, l'oxyde de zinc, l'oxyde de chrome III, l'oxyde de cuivre (II), l'oxyde d'aluminium (alumine).

On peut également citer comme oxydes mixtes, les zéolites ou tamis moléculaires gui sont des silicoaluminates, notamment sous leur forme silicoaluminates de métaux alcalins et préférentiellement de silicoaluminates de sodium ou de césium. Les zéolites de type faujasite conviennent tout particulièrement.

Le rapport molaire alcanal inférieur/diphénol peut varier largement. Généralement il se situe à des valeurs de 0,05 à 10.

De préférence le rapport molaire alcanal inférieur/diphénol est de 0,5 à 5.

La température à laquelle est conduit le procédé de l'invention se situe généralement entre 200°C et 500°C, à condition que le diphénol mis en oeuvre soit à l'état de vapeur.

De préférence la température de la réaction sera de 300°C à 450°C.

Dans cette zone préférée de température, gui convient de manière générale à tous les oxydes métalliques, il est encore plus favorable d'opérer dans des intervalles de températures adaptés selon l'oxyde métallique utilisé comme catalyseur.

Ainsi on préfère généralement réaliser le procédé entre 300°C et 350°C lorsque le catalyseur est un oxyde métallique acide ou amphotère et entre 350°C et 450°C lorsque le catalyseur est un oxyde métallique basique.

La réaction est habituellement conduite en continu dans un réacteur tubulaire comportant le catalyseur solide disposé en lit fixe.

Le catalyseur peut se présenter sous différentes formes : poudre, notamment dans les essais de laboratoire, ou produits mis en forme tels que granulés (par exemple des cylindres) billes, pastilles ou monolithes (blocs en forme de nid d'abeilles) qui sont obtenus par extrusion, moulage, compactage ou tout autre type de procédé connu.

Le diphénol et l'alcanal peuvent être introduits séparément ou en mélange dans le réacteur.

Ils peuvent également être introduits avec un diluant inerte qui est un solvant du diphénol et de l'alcanal.

A titre d'exemples non limitatifs de tels solvants, on peut citer les éthers et l'eau.

Parmi les éthers, on mentionnera plus particulièrement les éthers diméthyliques ou diéthyliques dérivant de l'oxyde d'éthylène ou de l'oxyde de propylène tels que le diméthyléther de l'éthylèneglycol (ou diméthoxy-1,2 éthane), le diéthyléther de l'éthylèneglycol (ou diéthoxy-1,2 éthane), le diméthoxy-1,5 oxa-3 pentane, le diéthoxy-1,5 oxa-3 pentane, le diméthoxy-1,8 dioxy-3,6 octane, le diméthoxy-1,11 trioxa-3,6,9 undécane, le diméthoxy-1,2 méthyl-1 éthane, le diméthoxy-1,5 diméthyl-1,4 oxa-3 pentane, le diméthoxy-1,8 triméthyl-1,4,7 dioxa-3,6 octane.

On peut également utiliser plusieurs solvants, comme par exemple un mélange d'eau et d'un éther tel que défini précédemment.

Il est généralement intéressant d'utiliser un gaz vecteur comme l'azote ou l'hydrogène ou un mélange des 2 gaz, pour faciliter le transfert des réactifs dans le réacteur.

Le temps de contact avec le catalyseur peut s'exprimer par le rapport entre le débit global (en volume/unité de temps) et le volume du catalyseur, à la température de réaction.

Le temps de contact est généralement de l'ordre de 0,1 seconde à 10 secondes.

On peut également utiliser la notion de productivité qui s'exprime par la quantité pondérale de réactif introduite par poids de catalyseur par unité de temps.

Les exemples qui suivent illustrent l'invention.

Les essais ont été réalisés dans un réacteur tubulaire vertical en verre Pyrex, de 15 mm de diamètre intérieur et de 20 cm³ de volume intérieur, équipé à sa partie supérieure d'une entrée latérale pour l'alimentation des fluides liquides et d'une entrée pour l'alimentation centrale des fluides gazeux, d'une gaine centrale de 5 mm de diamètre intérieur pour recevoir les thermocouples.

Le réacteur est muni à sa partie inférieure d'un disque en verre fritté, permettant de soutenir le catalyseur tout en laissant passer les fluides.

Dans le réacteur sont disposés successivement de bas en haut :

– une couche de 1 cm³ de poudre de verre (granulométrie : 2 mm) ;

– une couche constituée par le mélange de 3 cm³ d'oxyde métallique catalyseur (ayant une granulométrie de 0,8 à 1,25 mm) et de 6 cm³ de poudre de verre (granulométrie : 2 mm) ;

– une couche de 2 cm³ de poudre de verre (granulométrie : 2 mm).

Cet ensemble constitue le lit catalytique.

Il est également muni de moyens de chauffage du lit catalytique.

## EXEMPLES 1 à 5

Dans le réacteur décrit précédemment, différents essais sont effectués avec les oxydes métalliques indiqués dans le tableau ci-après.

Ces oxydes métalliques sont mis en oeuvre de la manière décrite précédemment.

On chauffe le lit catalytique à 440°C pendant 60 minutes, puis, tout en maintenant la température à 440°C, on injecte à l'aide d'un pousse-seringue, avec un débit de 4,37 g/heure, une solution préparée à partir de :

```
– hydroquinone                                : 2,75 g (25 mmol)

– trioxanne-1,3,5                             : 3 g (100 mmol)

– eau                                         : 0,45 g (25 mmol)

– éther diéthylique du diéthylène glycol  : 122 g (75 mmol).
```

On introduit également de l'hydrogène (sauf dans l'exemple 5 où le gaz vecteur est l'azote) avec un débit de 1,05 litre/heure (volume calculé dans les conditions normales de température et de pression).

On opère ainsi pendant 30 minutes pour établir le régime de l'appareil, puis pendant les 60 minutes suivantes le mélange réactionnel sortant du réacteur est piégé et est analysé par chromatographie en phase vapeur et par spectrométrie de masse.

Le temps de contact tel que défini précédemment est de 2 secondes pour chacun des essais.

Un essai témoin est effectué dans les mêmes conditions, mais le réacteur comporte alors uniquement de la poudre de verre (volume total de 12 cm³).

Le tableau ci-après rassemble les résultats obtenus.

| ESSAIS | CATALYSEUR | TT % HQ | RT % MeHQ | RT % Me2HQ | RT % MeBZQ |
|---|---|---|---|---|---|
| Témoin | billes de verre | 10 | 1 | 0 | 0 |
| Exemple 1 | Cr2O3 | 42 | 51 | 4 | 4 |
| Exemple 2 | Zr2O3 | 40 | 46 | 3 | 3 |
| Exemple 3 | La2O3 | 61 | 29 | 10 | 1 |
| Exemple 4 | MgO | 60 | 31 | 7 | 0 |
| Exemple 5 | MgO | 61 | 29 | 6 | 0 |

<u>Abréviations utilisées dans le tableau :</u>

- TT % HQ :      taux de transformation (en %) de l'hydroquinone
- RT % MeHQ :   rendement en méthylhydroquinone par rapport à l'hydroquinone transformée
- RT % Me2HQ : rendement en diméthylhydroquinone par rapport à l'hydroquinone transformée
- RT % MeBZQ : rendement en méthylbenzoquinone par rapport à l'hydroquinone transformée


**Revendications**

1. Procédé de C-alkylation de l'hydroquinone et de ses monoéthers, caractérisé en ce que l'on fait réagir en phase vapeur l'hydroquinone, ou l'un de ses monoéthers d'alkyle de 1 à 4 atomes de carbone ou de cyclohexyle, avec un alcanal inférieur en présence d'au moins un oxyde métallique.

2. Procédé selon la revendication 1, caractérisé en ce que l'on utilise comme monoéthers de l'hydroquinone, les monoéthers méthyliques, éthyliques, isopropyliques ou cyclohexyliques.

3. Procédé selon la revendication 1, caractérisé en ce que l'alcanal inférieur est choisi parmi le méthanal, l'éthanal, le propanal, le butanal.

4. Procédé selon la revendication 1, caractérisé en ce que l'alcanal est le méthanal.

5. Procédé selon la revendication 1, caractérisé en ce que les oxydes métalliques servant de catalyseurs sont des oxydes acides, amphotères ou basiques, simples ou mixtes ou des mélanges de ces différents oxydes.

6. Procédé selon l'une des revendications 1 à 5, caractérisé en ce que l'oxyde métallique acide ou amphotère, est choisi parmi les oxydes simples comme l'oxyde de silicium (silice), l'oxyde d'aluminium (alumine), le dioxyde de titane, le dioxyde de zirconium, l'oxyde stannique, l'oxyde arsénieux, l'oxyde de plomb II, l'oxyde de bismuth III, l'oxyde de vanadium V, l'oxyde d'antimoine V, le dioxyde de thorium, l'oxyde de chrome VI, l'oxyde de molybdène VI.

7. Procédé selon l'une des revendications 1 à 5, caractérisé en ce que l'oxyde métallique basique, est choisi parmi l'oxyde de magnésium, l'oxyde de lanthane, l'oxyde de zinc, l'oxyde de chrome, l'oxyde de cuivre, l'oxyde d'aluminium (alumine).

8. Procédé selon l'une des revendications 1 à 5, caractérisé en ce que l'oxyde métallique est choisi parmi les zéolites ou tamis moléculaires sous leur forme silicoaluminates de métaux alcalins et préférentiellement de silicoaluminates de sodium ou de césium.

4

9. Procédé selon la revendication 8, caractérisé en ce que les zéolites sont de type faujasite.

10. Procédé selon l'une des revendications 1 à 9, caractérisé en ce que le rapport molaire alcanal inférieur/diphénol se situe à des valeurs de 0,05 à 10 et de préférence de 0,5 à 5.

11. Procédé selon l'une des revendications 1 à 10, caractérisé en ce que la température à laquelle est conduit le procédé se situe entre 200°C et 500°C et de préférence entre 300°C à 450°C.

12. Procédé selon l'une des revendications 1 à 11, caractérisé en ce que la réaction est conduite en continu dans un réacteur tubulaire comportant le catalyseur solide disposé en lit fixe.

13. Procédé selon l'une des revendications 1 à 12, caractérisé en ce que le diphénol et l'alcanal sont introduits séparément ou en mélange dans le réacteur.

14. Procédé selon l'une des revendications 1 à 12, caractérisé en ce que le diphénol et l'alcanal sont introduits dans le réacteur avec un diluant inerte qui est un solvant du diphénol et de l'alcanal.

15. Procédé selon la revendication 14, caractérisé en ce que le solvant, est choisi parmi les éthers et l'eau.

16. Procédé selon l'une des revendications 14 et 15, caractérisé en ce que le solvant est choisi parmi les éthers diméthyliques ou diéthyliques dérivant de l'oxyde d'éthylène ou de l'oxyde de propylène tels que le diméthyléther de l'éthylèneglycol (ou diméthoxy-1,2 éthane), le diéthyléther de l'éthylèneglycol (ou diéthoxy-1,2 éthane), le diméthoxy-1,5 oxa-3 pentane, le diéthoxy-1,5 oxa-3 pentane, le diméthoxy-1,8 dioxy-3,6 octane, le diméthoxy-1,11 trioxa-3,6,9 undécane, le diméthoxy-1,2 méthyl-1 éthane, le diméthoxy-1,5 diméthyl-1,4 oxa-3 pentane, le diméthoxy-1,8 triméthyl-1,4,7 dioxa-3,6 octane.

17. Procédé selon l'une des revendications 1 à 16, caractérisé en ce que l'on utilise un gaz vecteur, comme l'azote ou l'hydrogène ou un mélange des 2 gaz, pour faciliter le transfert des réactifs dans le réacteur.

EP 0 438 329 A1

Office européen
des brevets

**RAPPORT DE RECHERCHE EUROPEENNE**

Numéro de la demande

EP 91 40 0030

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | FR-A-2 561 641 (RHONE-POULENC SANTE) * Revendication 1 * | 1 | C 07 C 37/20 C 07 C 39/08 |
| A | US-A-3 772 393 (HUNTER) * Colonne 4, lignes 55-63; colonne 6, exemple I * | 1,8 | |

DOMAINES TECHNIQUES RECHERCHES (Int. Cl.5)

C 07 C 37/00

Le présent rapport a été établi pour toutes les revendications

| Lieu de la recherche | Date d'achèvement de la recherche | Examinateur |
|---|---|---|
| LA HAYE | 16-04-1991 | KLAG M.J. |